**Europäisches Patentamt**

**European Patent Office**

**Office européen des brevets**

(19)

(11) Publication number : **0 484 107 A1**

(12) # EUROPEAN PATENT APPLICATION

(21) Application number : **91309987.5**

(22) Date of filing : **29.10.91**

(51) Int. Cl.⁵ : **A61B 5/0448,** A61B 5/0416, H01R 13/66, A61B 5/0428

(30) Priority : **30.10.90 US 605843**
**26.02.91 US 661253**
**07.10.91 US 772691**

(43) Date of publication of application :
**06.05.92 Bulletin 92/19**

(84) Designated Contracting States :
**AT BE CH DE DK ES FR GB GR IT LI LU NL SE**

(71) Applicant : **AMERICAN HOME PRODUCTS CORPORATION**
**685, Third Avenue**
**New York, New York 10017 (US)**

(72) Inventor : **Quedens, Phillipp John**
**51 Juniper Lane**
**Berlin, Connecticut 06037 (US)**

Inventor : **Boucher, Donald Roy**
**3 Hayledge Court**
**Wallingford, Connecticut 06492 (US)**
Inventor : **Shipherd, John Tilden**
**38 Beekman Place**
**Madison, Connecticut 06443 (US)**
Inventor : **Malis, Michael Jacob**
**61 Grayrock Road**
**Trumbull, Connecticut 06611 (US)**
Inventor : **Poirier, James Warren**
**1988 Middletown Avenue**
**Northford, Connecticut 06472 (US)**
Inventor : **Izzo, Joseph Anthony**
**16 Alfred Street**
**New Haven, Connecticut 06512 (US)**

(74) Representative : **Wileman, David Francis Dr. et al**
**c/o Wyeth Laboratories Huntercombe Lane South**
**Taplow Maidenhead Berkshire SL6 OPH (GB)**

(54) **Electrical connection device for use in monitoring fetal heart rate.**

(57) A connect or device for electrically connecting fetal and maternal electrodes (10, 12) with a plate support on the body of an expectant mother. A male connector (20) and a female connector (38, 40) engage each other so as to effect electrical connection therebetween. The male and female connectors releasably retain each other when the electrical connection is effected. One of the male and female connectors is electrically connected to the fetal and maternal electrodes. The plate support includes a housing (32) supporting the other of the male and female connectors. The housing may have a passage (34) in which is engaged the male and female connectors.

Fig. 1.

EP 0 484 107 A1

The present invention relates to a connector device for use in monitoring fetal heart rate. The device electrically connects fetal and maternal electrodes to a plate support on the body of an expectant mother. A remote fetal monitoring device may then be electrically connected to the electrodes via the connector device.

U.S. Patent No. Re. 28,990, which is incorporated herein by reference, discloses the bipolar fetal electrode assembly commonly used to monitor fetal heart rate during birth. In the use of that assembly, a doctor inserts the forward end of a curved guide tube through the mother's vagina and cervix until the forward end of the guide tube makes contact with the fetal head or other portion of the fetus. Holding the forward end of the guide tube stationary, the doctor then pushes the rear end of a flexible driving tube forwardly until a spiral fetal electrode at the forward end of one wire of a twisted wire pair makes contact with the fetal epidermis. The forward end of the other wire has a spade-like maternal electrode which is electrically isolated from the spiral fetal electrode.

The doctor then rotates the flexible driving tube clockwise about one full turn while maintaining the forward end of the guide tube against the fetal head. This will screw the spiral electrode into the fetal epidermis. Thereafter, the doctor removes his fingers from the mother's vagina, grasps the outer ends of the driving tube and the guide tube, and slides these tubes as a unit off the wires, leaving only the bipolar electrodes and the two twisted wires within the mother.

The outer ends of the wires are then connected to a suitable apparatus for monitoring fetal heart rate. Such an apparatus is discussed in U.S. Patent No. 4,632,121, the contents of which are also incorporated by reference and which also shows a cable assembly for effecting electrical connection between the electrodes and the fetal monitor. A galvanic potential difference may then be measured between the bipolar electrodes. The wires connected to the electrodes are twisted about each other so that any induced voltages caused by external electromagnetic interference will be the same in each and therefore will not adversely affect the measurement of the galvanic potential difference between the electrodes.

In practice, the ends of the twisted wires are left uninsulated, e.g. by as much as 5/8 inch to 3/4 inch, to allow connection to the monitor and to enable removal of the guide and driving tubes from the twisted wires.

Manually connecting the uninsulated ends of the twisted wire pair to the base plate is somewhat cumbersome and creates the possibility that the wires may unintentionally short each other. If shorted, the wires will be unable to transmit correct signals from the fetal and maternal electrodes.

It would therefore be desirable to effect electrical connection of the fetal and maternal electrodes with a remote fetal monitoring device without handling uninsulated ends of wires.

The present invention is directed to a device for electrically connecting fetal and maternal electrodes to a plate support on the body of an expectant mother.

According to this invention there is provided a connector device for electrically connecting fetal and maternal electrodes to a plate support on the body of an expectant mother, comprising:

a housing of the plate support;

electrical connection means for making electrical connection between a remote fetal monitoring device and the fetal and maternal electrodes the connection means including input and output means which are electrically connected together within the housing, the input means including a male connector and a female connector adapted to engage each other for establishing electrical connection therebetween, the one of the male connector and the female connector being electrically connected with the fetal and maternal electrodes, the other of the male connector and the female connector being electrically connected with the output means, the output means being adapted for making electrical connection with the remote fetal monitoring device; and

releasable retaining means for releasably retaining the male and female connectors together when the electrical connection is established between the male and female connectors.

The device preferably includes a female connector and a male connector which engage each other to effect electrical connection therebetween. One of the connectors is electrically connected to the fetal and maternal electrodes and the other is electrically connected to the remote fetal monitoring device. The connectors are releasably retained with each other for maintaining the electrical connection.

Preferably the one of the male and female connectors has two terminals electrically isolated from each other and each in electrical connection with a respective one of the fetal and maternal electrodes.

For example the connection means within the housing can be a male or female connector having two contacts electrically isolated from each other and in electrical connection with the output means, the male or female connector being adapted to make slidable and releasable electrical connection via an appropriate female or male connector to the fetal and maternal electrodes.

Preferably the connector device has a base supporting a ground electrode for electrical connection with the mother, and means for electrically connecting the ground electrode to the output means and for securing the base and housing together.

This invention also provides a housing device for electrically connecting fetal and maternal electrodes with a plate support on the body of an expectant. mother, comprising:

a housing of the plate support;

electrical connection means for making electrical connection between a remote fetal monitoring device and the fetal and maternal electrodes, the connection means including a male or female connector and an output means which are electrically connected together within the housing, the male or female connector having two contacts electrically isolated from each other and in electrical connection with the output means said male or female connector being adapted to make slidable and releasable electrical connection with the fetal and maternal electrodes, the output means being adapted for making electrical connection with the remote fetal monitoring device.

In a preferred embodiment the devices have a printed circuit board within the housing, the printed circuit board having conductive tracers which are part of the electrical connection means.

It is also preferred that the devices are provided with conductive test terminals which are accessible from outside the housing, the test terminals being in electrical connection with respective ones of the conductive tracers.

Further provided by this invention is an electrode device for electrically connecting fetal and maternal electrodes to a plate support on the body of an expectant mother, the plate support having a housing with a male or female connector, the device comprising:

fetal and maternal electrodes; and

electrical connection means for making electrical connection between a remote fetal monitoring device and the fetal and maternal electrodes, the connection means including a connector electrically connected with the fetal and maternal electrodes, the connector having a body with two contacts electrically isolated from each other, each of the contacts being in electrical connection with a respective one of the electrodes.

Preferably the fetal/maternal electrode device further comprises a hollow handle secured to a driving tube, the handle widening in diameter as the handle extends away from the driving tube and the connector has a tapered forward end so as to reduce the risk of jamming when inserted in the hollow handle.

BRIEF DESCRIPTION OF THE DRAWINGS

For a better understanding of the present invention, reference is made to the following description and accompanying drawings, while the scope of the invention is set forth in the appended claims.

Fig. 1 shows a perspective view of a first embodiment of a connector device for use in monitoring fetal heart rate in accordance with the invention.

Fig. 2 shows a cross-sectional view across section lines 2-2 of Fig. 1.

Fig. 3 shows an exploded perspective view of the leg plate assembly of the connector device of Fig. 1.

Fig. 4 shows a perspective view of the connector of Fig. 1 and the printed circuit board of Fig. 3.

Fig. 5 shows a cross-sectional view across section lines 5-5 of Fig. 4 after insertion of the connector into the opening in the housing of the leg plate.

Fig. 6 shows a schematic view of the printed circuit board of Fig. 3 in accordance with the invention.

Fig. 7 shows a top view of a second embodiment of the connector device in accordance with the invention.

Fig. 8 shows a perspective view of the base of Fig. 7 with the leg plate housing of Fig. 1 over the base. A clip for the belt is a variation of the clip of Fig. 7.

Fig. 9 shows a cross-sectional view across section lines 9-9 of Fig. 7 after assembly of the housing onto the base.

Fig. 10 shows a perspective view of the leg plate housing of Fig. 1 and a test adapter in accordance with the invention.

Fig. 11 shows a rear elevation view of the test adapter depicted in Fig. 10.

Fig. 12 shows a cross-sectional view across section lines 12-12 of Fig. 13 with the leg plate housing shown inserted into a test adapter.

Fig. 13 shows a partial cross-sectional view across section lines 13-13 of Fig. 12.

Fig. 14 shows a perspective view of a third embodiment of the invention in which the attachment of the belt to the housing varies from the other embodiments and is shown arranged on a leg of a patient.

Fig. 15 shows a partially broken side elevation view of the embodiment of Fig. 14.

Fig. 16 is a front end view of the leg plate in accordance with another embodiment which has an "O" ring seal.

Fig. 17 is a partial longitudinal cross-sectional view of Fig. 16.

Fig. 18 is a longitudinal cross-section of the electrode assembly showing the guide and driving tubes being removed from the connected electrodes.

Figure 19 is a perspective view of the leg plate and female connector prior to engagement in accordance with a flat side embodiment of the present invention.

Figure 20 is a perspective view of the female connector of Fig. 19.

Figure 21 is an end view of Fig. 20.

Figure 22 is an end view of the leg plate of Fig. 19.

Figure 23 is a perspective view of the leg plate and female connector prior to their engagement in accordance with another flat side embodiment of the present application.

Figure 24 is perspective view of the leg plate and female connector prior to their engagement in accordance with a ring contact embodiment of the present invention.

## DESCRIPTION OF THE PREFERRED EMBODIMENT

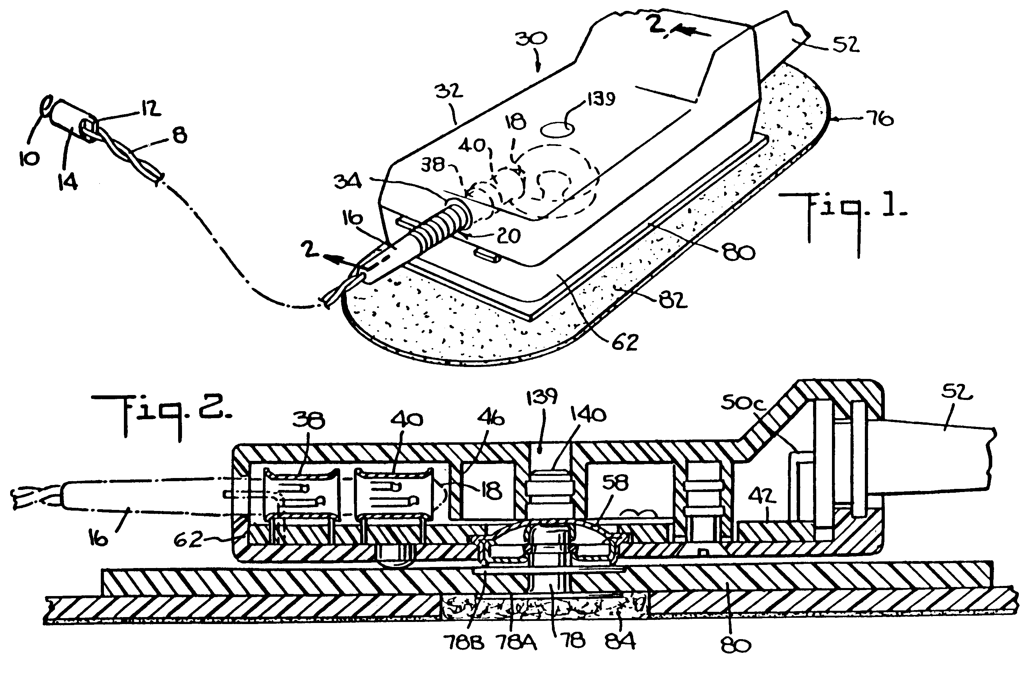

Fig. 1 shows a conventional fetal electrode product (in accordance with U.S. Patent No. Re. 28,990) which includes a twisted pair of wires 8 whose ends are connected to respective fetal and maternal electrodes 10, 12. A nonconductive plastic holder 14 electrically insulates the fetal and maternal electrodes 10, 12 from each other. The twisted wire pair 8 extends through a finned thermoplastic strain relief element 16 into a connector 20 in accordance with the invention.

The fetal electrode 10 is in the form of a spiral electrode having a pointed end which is driven into contact with the fetal epidermis. The maternal electrode 12 is engageable by slots (not shown) at the forward end of a driving tube (not shown in Fig. 1) to enable the pointed end of the fetal electrode 10 to rotate and be driven into the fetal epidermis by rotation of the driving tube (not shown in Fig. 1).

Referring to Figs. 1 and 4, the connector 20 has a forward tapered tip 18 and two electrically conductive terminals 22, 24. The terminals 22, 24 are spaced from each other and attached to the respective proximal ends 26, 28 of the twisted wire pair 8. A leg plate 30 has a housing 32 with an opening 34 in which is inserted the connector 20.

Within the opening 34 are two fingers 38A of a front barrel contact 38 and two fingers 40A in a rear barrel contact 40. The two fingers 38A are diametrically opposite each other, as are the two fingers 40A. The barrel contacts are each cylindrical and mounted on a printed circuit board (PCB) 42 and have open ends in line with each other. Barrel contact 38 is between the entrance of the opening 34 and the other barrel contact 40. The front and rear barrel contacts are identical and constitute input contacts.

Each of the two fingers 38A, 40A have respective free ends which are convexly curved. Upon full insertion of the connector 20 through the open ends of both barrel contacts 38 and 40, the free ends of the fingers 38A, 40A respectively engage the terminals 22, 24 and exert about a quarter pound force on the connector because the diameter of the connector is wider than the inner diametrical width defined between the diametrically spaced apart fingers 38A, 40A when the fingers are in an untensioned state.

The barrel contact 40 also has a pair of two diametrically opposite fingers 40B each with a convexly curved free end that biases into contact with the terminal 24 of connector 20 upon full insertion of the connector 20 into the barrel contacts 38, 40. The barrel contact 38 also has a pair of two diametrically opposite fingers 38B each with a convexly curved free end that biases into contact with a recess 44; that is, this free end clicks into place in the recess. The recess 44 is curved and extends about the outer circumference of the connector 20.

This clicking into place of fingers 38B with recess 44 causes a sudden change in resistance to further advancing movement of the connector in the leg plate and is noticeably felt by the person who is inserting the connector. This signifies to such person that full insertion has been attained and that electrical connection between the terminals 22, 24 and the barrel contacts 38, 40 has been established.

As further confirmation that the connector 20 has been fully inserted, an interior wall 46 of the housing 32 (see Fig. 2) is arranged directly at the end of the path of insertion of the connector 20. When the tapered tip 18 of the connector 20 (see Figs. 2 and 4) contacts this wall 46, it is effectively blocked by the wall 46, thereby also signifying full insertion of the connector 20 into the barrel contacts 38, 40 and preventing any further advance of the connector 20 into the leg plate.

As described, it may be appreciated that the connector 20 is a male type and the opening 34 with barrel contacts 38, 40 constitutes a female type connector. Thus, the male is inserted into the female for establishing electrical connection therebetween.

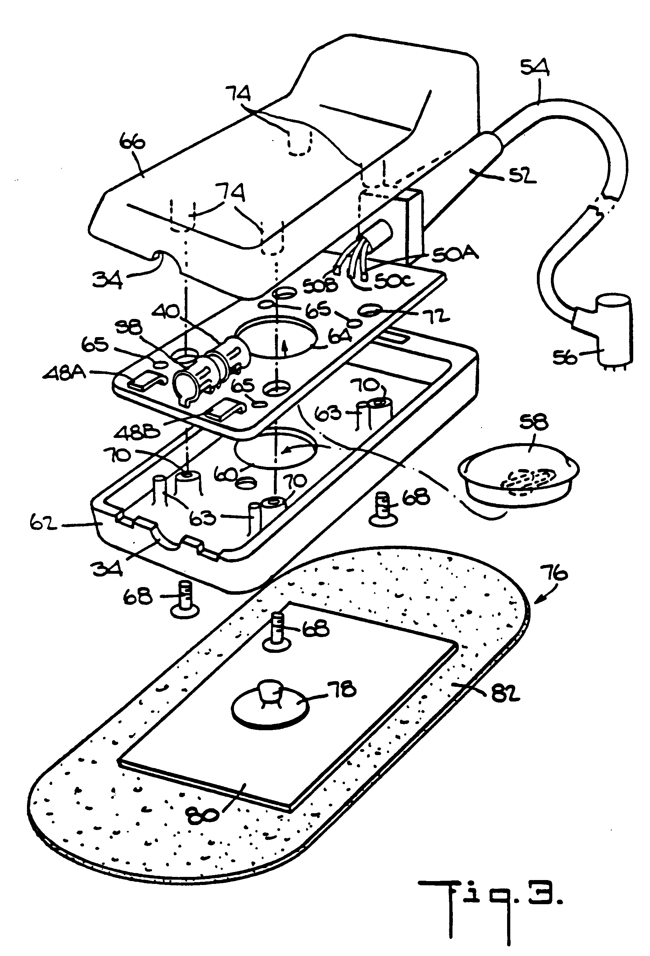

Fig. 3 is illustrative of the method of assembly of the leg plate. The barrel contacts 38, 40, the test terminals 48A, 48B and 48C (see Fig. 4), and the three output leads 50A, B, C are all soldered onto PCB 42. The output leads 50A, B, C extend from PCB 42 through shielded cable 54, which leads to a plug 56. After assembly is completed, the output leads 50a, 50b, and 50c extend from PCB 42 through strain relief 52 via a shielded cable 54 and plug 56 to the fetal monitor.

Next, a snap compartment 58 is secured within an opening 60 in the bottom half 62 of the housing or in the opening 64 in the PCB 42. If the snap compartment 58 is secured to the PCB 42, care must be taken to avoid fracture of the PCB 42 from stresses acting on the PCB during snapping engagement.

The housing bottom and top halves 62, 66 are brought together to enclose the PCB 42. The PCB 42 is secured to the bottom housing 62 by ultrasonic staking the four plastic studs 63 after they go through respective mating holes 65 in PCB 42. Staking involves melting the ends of the plastic studs ultra-sonically onto the PCB 42, which ensures that alignment of opening 34 with contacts 38, 40 is permanent. The stems of four screws 68 are inserted through respective hollow bosses 70 in the bottom half 62 and through respective holes 72 in the PCB 42 that are aligned with the hollow bosses 70. The stems of the screws 68 are then screwed into threaded bosses 74 which extend from the underside of the upper half 66. In this manner, the housing halves 62, 66 become secured with each other. Test terminals 48A, 48B and 48C protrude out of the housing.

The assembled housing 32, which has a rectangular bed-like shape as seen in Fig. 1, is then ready to be secured to the base 76 by engaging a snap stud 78 into the opening in the snap compartment 58. The snap stud 78 may be considered a ground electrode and has two plate portions 78A, 78B (see Fig. 2). Between the plate portions 78A,78B is held a center portion of a vinyl board 80. An adhesively coated flexible foam pad 82 is secured to the underside of the vinyl board 80 and has a central circular opening beneath the plate portion 78A. A conductive gel 84 is arranged in this central circular opening and is mixed with a reticulated foam.

The vinyl board 80 is rectangular as is the bottom surface of the bottom half 62 of the housing 32. When the sides of each are aligned with each other, the snap stud 78 is aligned with the receiving hole of the snap compartment 58. Effecting snapping engagement between the housing 32 and the base 82 when the base 82 is secured to the patient's leg may be done readily by aligning the sides of the lower housing half 62 with the sides of the vinyl board, which in turn aligns snap compartment 58 with the snap stud 78. The size of the surfaces of the vinyl board and bottom housing half 62 which face each other need not be the same size; the size of the surface of the vinyl board may be slightly larger and still be able to be a guide for alignment to effect snapping engagement. Further, these surfaces need not be rectangular, but it is preferred that they conform in shape with each other.

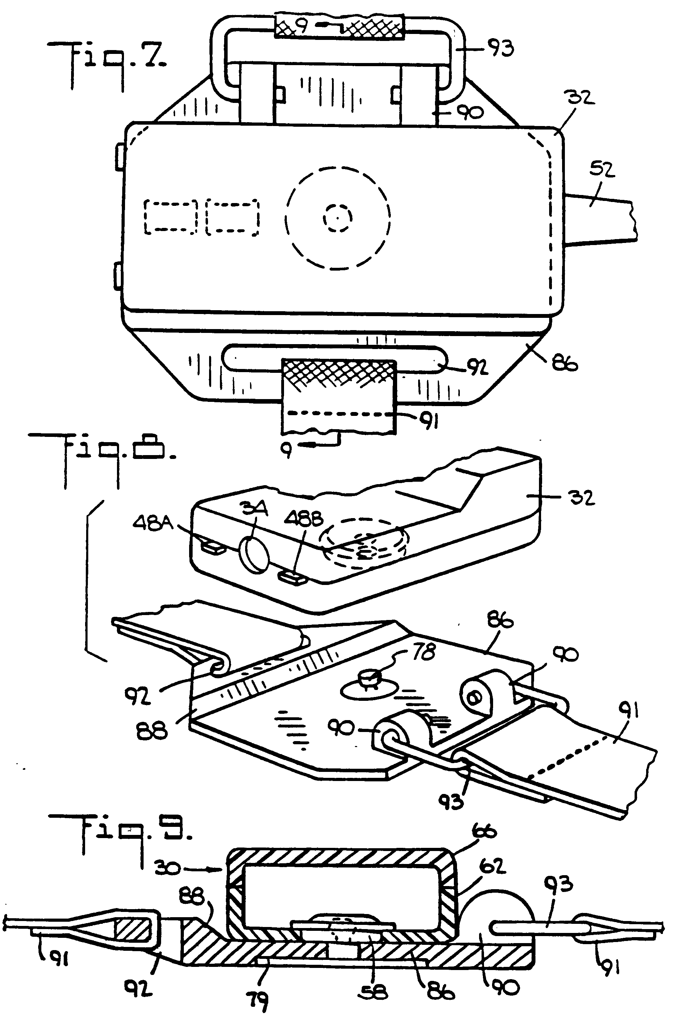

Figs. 7, 8 and 9 show an alternative embodiment in which the same assembled housing 32 is secured to a different base 86, which has its own snap stud 78 for effecting snapping engagement in a like manner as that for base 76 of Fig. 3. Projection 88 extends upward from the base 86 adjacent to a side of the housing 32 so as to block relative rotation of the housing 32 on the snap 78 by blocking the corners of the housing.

Belt clasp holders 90 are provided on one side of the base 86 and a slot 92 is provided on the opposite side of the base 86. A belt 91 has an end which is looped through the slot 92 and is wrapped around the expectant mother's leg or abdominal region. Fig. 8 shows a clasp having free ends inserted through respective aligned holes in the belt clasp holders 90 and has a central segment inserted through a loop at another end of the belt. The free ends may point inwardly as shown or else outwardly. In both cases, the clasp may be resiliently bendable plastic or else made of rigid metal and be permanent.

The belt holders 90 also block relative rotation of the housing 32 on the base 86 because they project upward from the base 86 adjacent to another side of the housing 32. Together, projection 88 and belt holders 90 serve as guides for placement of the housing 32 on the base 86 by defining an area therebetween on the base 86 for receiving the housing. Preferably, this area conforms in shape to the underside of the housing bottom half 62.

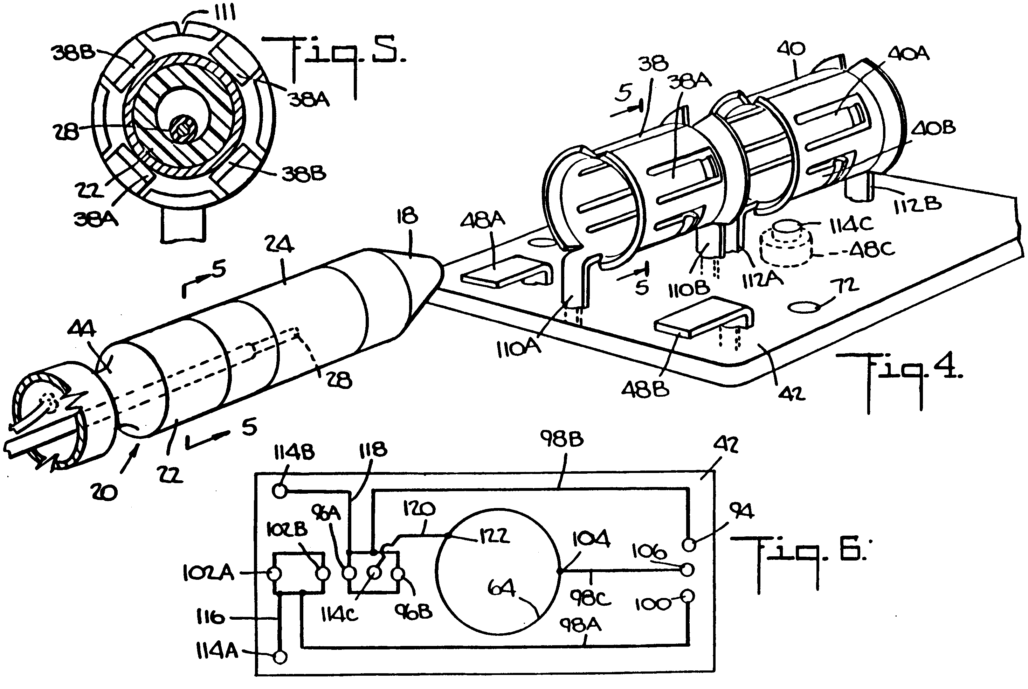

Fig. 6 shows that the printed circuit board 42 has three conductive tracers 98A, 98B, 98C. The first conductive tracer 98A extends between output terminal 100 and input terminals 102A, 102B. The second conductive tracer 98B extends between output terminal 94 and input terminals 96A, 96B. The third conductive tracer 98C extends between ground ring terminal 104 and output terminal 106.

Referring to Figs. 3, 4 and 6, it can appreciated that the three output leads 50A, B, C are respectively soldered to output terminals 100, 94 and 106. Two bent legs 110A, 110B of barrel contact 38 are respectively soldered to input terminals 102A, 102B. Two bent legs 112A, 112B of barrel contact 40 are respectively soldered to input terminals 96A, 96B.

The legs of the barrel contacts 38, 40 are subject to fatigue from vibrations caused by repetitive insertion and removal of the connector 20 in the barrel contacts 38, 40. By having both legs in electrical contact with a tracer, electrical connection is maintained as long as at least one leg remains unbroken and in electrical connection. Each barrel contact should be able to withstand 5000 cycles of insertion and removal of the connector 20 without breakage.

The barrel contacts 38, 40 are identical to each other for sake of economy in manufacture. Fabrication of the barrel contacts 38, 40 involves feeding a sheet of metal, such as beryllium copper, through a progressive stamping die, which stamps the sheet metal into its final shape. The stamped sheet is then heat treated by heating for strengthening.

For barrel contact 38, for instance, cut outs are made in the metal sheet by the progressive stamping die to define the fingers 38A, B and the legs 110A, B. Next, the free ends of the fingers are convexly curved to project from one face of the sheet and the legs are bent to project from the opposite face. The sheet is then bent into a cylindrical shape as shown in Fig. 4 to form a hollow barrel contact whose ends are brought together to form a V-like space 111 as shown at the top of Fig. 5. The fingers project inwardly and the legs project outwardly. The barrel contacts are gold plated for providing corrosion and wear resistance. The fingers 38A,B and 40A,B are resilient so that their convexly curved free ends bias inwardly when forced outwardly upon insertion of the connector 20 into the barrel contacts 38, 40.

Of course, it is not actually necessary for barrel contact 40 to have two sets of pairs of fingers 40A, 40B; either pairs of fingers 40A or pairs of fingers 40B will suffice for establishing contact with terminal 24. However, if the barrel contacts are identical, the same progressive stamping die may be used.

After insertion of the connector 20 into the opening 34, electrical contact is established between the terminals 22, 24 and the barrel contacts 38, 40, respectively. In this manner, an electrical circuit path is made between the fetal electrode 10, one respective twisted wire 8, terminal 22, barrel contact 38, tracer 98A and output terminal 100. An electrical circuit path is also made between the maternal electrode 12, the other twisted wire 8, terminal 24, barrel contact 40, tracer 98B and output terminal 94. Another electrical circuit path is made between the snap stud 78, the snap compartment 58, the ground ring terminal 104, the tracer 98C and the output terminal 106. The ground ring terminal 104 extends about the circumference of the opening 64 to form a ring and is covered by solder.

Preferably, the output leads 50A, B, C are color coded; e.g., the lead 50B from output terminal 94 may be blue, the lead 50A from output terminal 100 may be red and the lead 50C from output terminal 106 may be white. In this manner, the blue lead 50B is associated with the maternal electrode 12; the red lead 50A is associated with the fetal electrode 10; and the white lead 50C is associated with the ground electrode, e.g., plate portion 78A. This helps to ensure recognition of the leads from each other during assembly of plug 56.

It should be appreciated that the construction of the connector and leg plate as described helps to avoid the possibility of inadvertently mixing up interconnecting wires, and does so without relying on color coding (other than for the output leads). Since the tracer terminals lie directly beneath the components to which they are to be electrically connected, the potential for confusion in making the correct electrical connection therebetween is obviated.

Further, correspondence between the output leads and the spiral and maternal electrodes is assured by maintaining the relative position of the terminals themselves. Since the fetal electrode 10 is arranged further forward at the forward wire end 2 than is the maternal electrode 12, the connector terminal 22 is arranged further forward towards the forward wire end 2 and closer to the fetal electrode 10 than is connector terminal 24. The correspondence between the fetal and maternal electrodes and their respective connector terminals thereby becomes evident by inspection without the need for color coding the twisted wire pair.

In addition, the relative arrangement of the barrel contacts 38, 40 with respect to each other is arranged in a like manner, i.e., barrel contact 38 is further forward (closer to the entrance of the opening 34) than is barrel contact 40. Thus, when the connector is inserted into barrel contacts 38, 40, the first barrel contact which the connector passes through is barrel contact 38. After full insertion of the connector 20 in the opening 34, the connector terminal 22 makes electrical connection with barrel contact 38, thereby associating barrel contact 38 with fetal electrode 10. Similarly, barrel contact 40 becomes associated with maternal electrode 12.

Conductive tracers 98A, 98B extend from their respective connection with the barrel contacts 38, 40 to terminate at the respective output terminals 100, 94, which are spaced from either side of output terminal 106. Thus, the association between the output terminals and the respective electrode can be readily made by referring to Figs. 4 and 6 which illustrate the connections between the output terminal and the barrel contact.

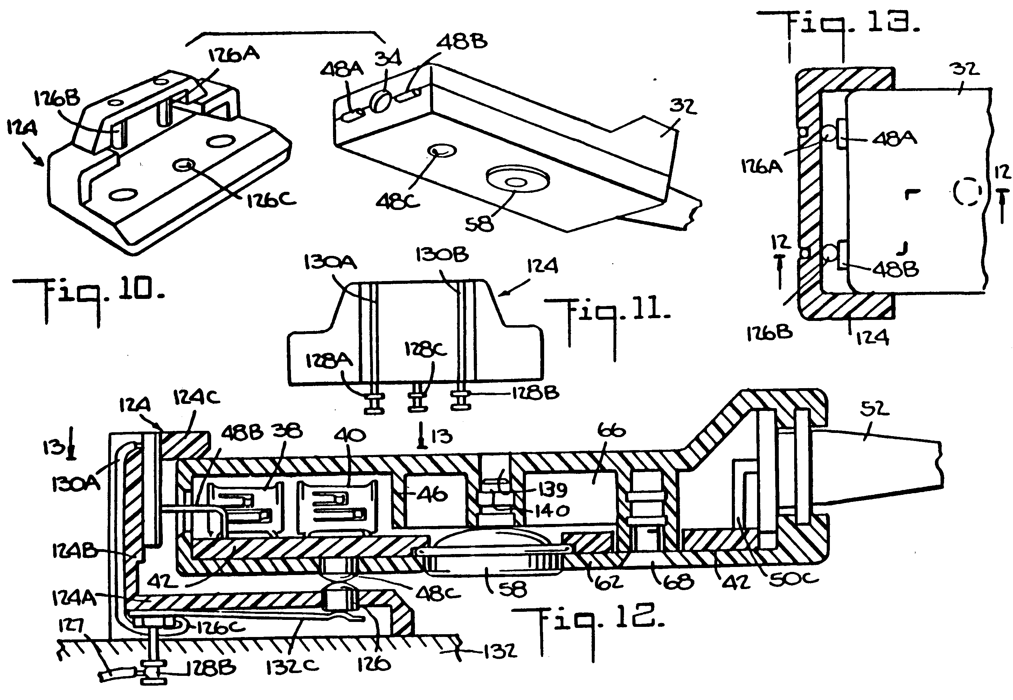

Figs. 10-13 show a connector device for testing purposes in which test terminals 48A, 48B and 48C are electrically connected, respectively, with the barrel contacts 38, 40 and snap compartment 58. Test terminals 48A, 48B, 48C are soldered to respective test contacts 114A, 114B, 114C. The test tracer 116 extends between input terminals 102A, 102B and the test contact 114A. The test tracer 118 extends between input terminals 96A, 96B and the test contact 114B. The test tracer 120 extends between test input terminal 122 and the test contact 114C. Test input terminal 122 is part of ground ring terminal 104 and thereby electrically connects test contact 114C and tracer 120 with the snap compartment 58 and compartment seat 64.

Test terminals 48A and 48B project from one end of the housing 32 and test terminal 48C projects from the underside of the housing 32. The end of the housing with the test terminals 48A, B is inserted, for test purposes, into an adapter 124 attached to the rear of a fetal monitor. As shown, the opening 34 is free of the connector 20.

The adapter 124 has a long side 124A, an end side 124B, and a short side 124C. The long and short sides 124A, C are substantially parallel to each other and the end side 124B extends therebetween. The end side of the housing with the test terminals 48A, 48B is against the end side 124B of the adapter 124 for enabling the test terminals 48A, B to make electrical connection with adapter contacts 126A, 126B, respectively. The housing is retained in place in the adapter by gravity. The top housing 66 is guided against an inner facing surface of the short side 124A while the test terminal 48C presses into contact with the adapter contact 126C protruding inwardly from the long side 124A.

The adapter contacts 126A, B, C are in respective electrical connection with contact pins 128A, B, C at the underside of the adapter 124 via respective conductive elements 130A, B, C. The rear side of the fetal monitor has three mating terminals 127 (only one shown) which mate respectively with the three contact pins 128A, 128B, 128C as the housing is inserted into the adapter 124.

A simulated R-wave signal is transmitted from the fetal monitor via the mating terminals 27. This simulated R-wave signal is typical of an EKG or ECG signal and has approximately 100 microvolt peak-to-peak amplitude at a rate of 120 beats per minute.

In so doing, three test paths may be checked. The three test paths include the respective test terminals 48A, B, C; tracers 116, 118, 120; tracers 98A, 98B, 98C; output terminals 100, 94, 106; output leads 50A, B, C; cable 54; plug 56; fetal monitor electronics (not shown), a monitor display (not shown), and a monitor chart recorder (not shown).

If any of the three test paths are open, the monitor will not count the beat of the simulated R-wave that should be received by the fetal monitor after its transmission. This is because either there is no signal being developed at an amplifier input of the fetal monitor or there is a return path open downstream of the amplifier input which causes the amplifier input to saturate.

An operator of the fetal monitor recognizes the count signal if the leg plate passes the test. The leg plate connector and fetal monitor may then be used since the possibility of equipment malfunction is ruled out. Otherwise, if the count can not be recognized, servicing is required.

If the leg plate connector passes the test and the connector device still does not function properly, then the cause for the problem at least has been somewhat isolated, i.e., the problem is either that the grounding is poor, stray capacitance is affecting the twisted wire pair too much, or the barrel contacts are too dirty or their legs have broken. The grounding may be improved by adding more conductive gel 84. The stray capacitance problem may be remedied by replacing the twisted wire pair and connector. If the legs of the barrel contacts have broken, then servicing is required.

The contacts 38, 40, 58 may be used instead of the associated test leads 48A, B, C for test purposes by modifying the adaptor so that any of its adapter contacts 126A, B or C are repositioned and project further outwardly to effect electrical connection with any of the contacts 38, 40 or 58, respectively.

The adapter 124 may be further modified to accommodate insertion of the housing with its base attached. In this case, either a ground test terminal extends through the base in contact with the test lead 48C or the ground electrode in the base itself is used for electrically connecting with the adapter contact 126C. The ground electrode itself may be used in this manner preferably only if the conductive gel 84 is to be added later.

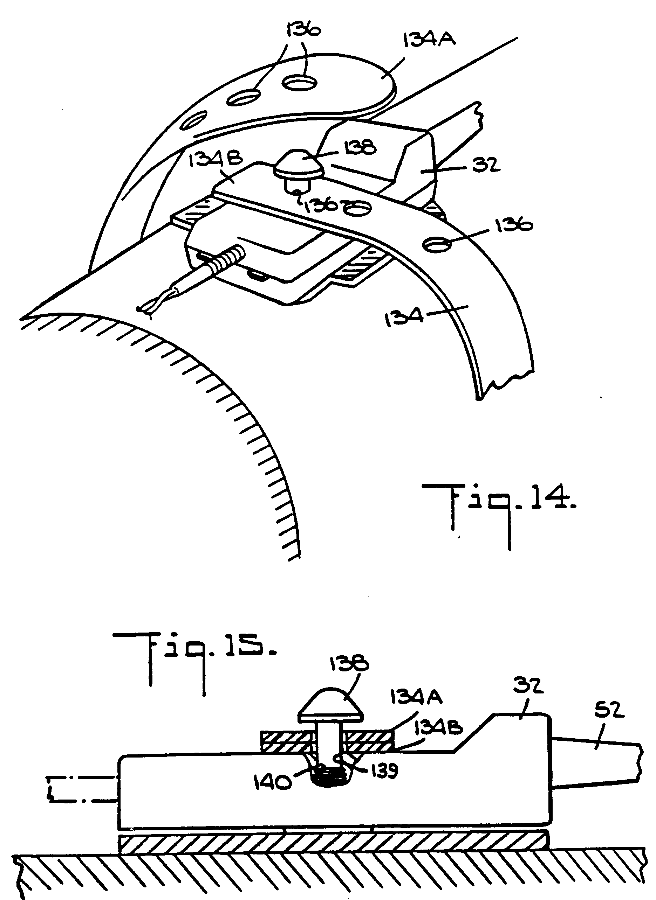

Figs. 14 and 15 show another embodiment similar to that of Fig. 8, except that a buttonhole belt 134 with button holes 136 is wrapped around a patient's leg. One hole at each end 134A, 134B of the belt 134 is fitted onto a mushroom hat 138. The hat protrudes from the housing 32 from a hole 139 and is threadably engaged with a threaded socket 140 in the housing 32. The diameter of the holes is slightly larger or about the same size as the widest diameter of the hat 138. The bottom of socket 140 is insulated to prevent electrical connection with the snap compartment 58.

Figs. 16-17 show an optional arrangement which may be used for any of the other embodiments. The entrance of the opening 34 of the housing 32 is modified to provide a chamber 142 in which is arranged an O-ring seal 144, which made of an elastic material. The tapered tip 18 of the connector 20 is inserted through the inner space defined by the O-ring seal 144 in order to reach the barrel contacts 38, 40. As the tapered tip 18 is inserted, the O-ring seal 144 is compressed outward and follows the diameter of the connector 20, thereby wiping the outer surface of the connector 20 clean of contaminants. At the recess 44, the O-ring seal 144 is free to expand again to its relaxed state but is again compressed outward as the connector 20 is inserted further into the opening 34. By virtue of the O-ring seal 144 rubbing the outer surface of the connector 20, contaminants that are on the connector 20 are prevented from contaminating the barrel contacts 38, 40 in the opening 34.

It should be understood that the engagement between the recess of the connector and the finger of the barrel contact may be made instead by providing the connector with the finger and the barrel contact with the recess. In addition, analogous components of any embodiment are interchangeable with those from the other embodiments or those found in U.S. Patent Application Serial No. 605,843.

Further, the finger 38A on barrel contact 38 alone could serve not only for engaging with the recess 44, but also serve to effect electrical connection with terminal 22 where terminal 22 is arranged within the recess 44.

It is preferred, however, that an identical recess to that of recess 44 be added to the connector 20 between terminals 22 and 24 to enable the fingers 38A, 40A to click into place in both recesses simultaneously. This is preferred for keeping the unit held together during the fabrication process. Thus, when used, a user would have to listen for two clicks before knowing that full insertion had been made. That is, the first click arises from finger 38A entering into the newly added recess and the second click arises from the simultaneous click of finger 38A entering into recess 44 and finger 40A entering into the newly added recess.

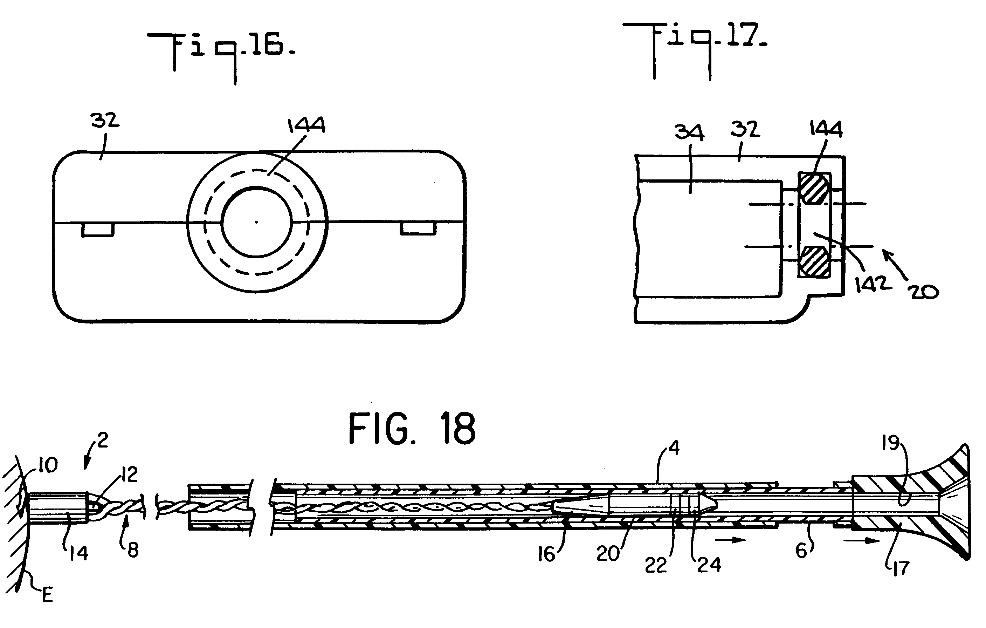

Fig. 18 shows the manner of removal of a guide tube 4 and a driving tube 6 from the connector 20 and twisted wire pair 8. The spiral electrode 10 is in contact with the fetal epidermis E. The tubes 4,6 are removed by pulling the driving handle 17 and guide tube 4 in the direction indicated by the direction arrows, i.e., in a direction away from the fetal and maternal electrodes 10, 12. After removal of the tubes, the connector 20 is accessible for its insertion into a leg plate.

The drive handle 17 defines a passage 19 in communication with the inner passage defined by the driving tube 6. The driving tube 6 is jam fit into handle 17 along an inner taper of handle 17 that extends toward a stepped surface of the handle 17.

Handle 17, which has a cone-like shape facing rearwardly, facilitates pulling of the driving tube 6 off the twisted wire pair 8. The distal end 16 of the connector 20 is tapered to fit within the open end of handle 17 and thereby travel within the driving tube 6 without jamming.

Preferably, the electrodes and electrical contacts and conductors are made of nickel or gold plated copper or else gold plated over nickel plated copper. High density polyethylene (such as 12 melt Arco #7120 or Chevron #9160 or petrothene LS 606 8 melt, density 0.96) is recommended as the material for the guide and driving tubes 4, 6 handle 17 and the housing 32. The conductors are preferably jacketed with polyvinylchloride. All other components may be made from the same materials as their counterparts in the U.S. Patent No. Re. 28,990.

The outer diameter of the guide tube 4 must be small enough to avoid harming the mother during its insertion, such as about 0.315 inches. The inner diameter of the driving tube 6 may be about 0.146 inches. Thus, the width of the connector 20 must be smaller to fit within the driving tube 6.

The male connector 20 may be replaced by a female connector type, in which case the leg plate would have a male type connector, which may either protrude outward from the leg plate or be enclosed to extend within a passage in the leg plate.

Such a replacement is advantageous in the case where the male type connector may be inserted by mistake into a wall outlet. Such an insertion poses a safety hazard where the male type connector is electrically connected to the fetal and maternal electrodes within the expectant mother. By replacing the male type connector with a female type connector for connection with the electrodes, this potential safety hazard is eliminated. The female connector has its body surrounding its terminals so that electrical connection can not be effected between the female connector and the wall outlet because the female connector does not have contacts accessible from its outer periphery.

The male connector and the female connector each have two connector terminals. When the female connector shrouds the male connector, its two terminals each make electrical contact with a respective one of the two terminals of the male connector and the two connectors releasably engage each other.

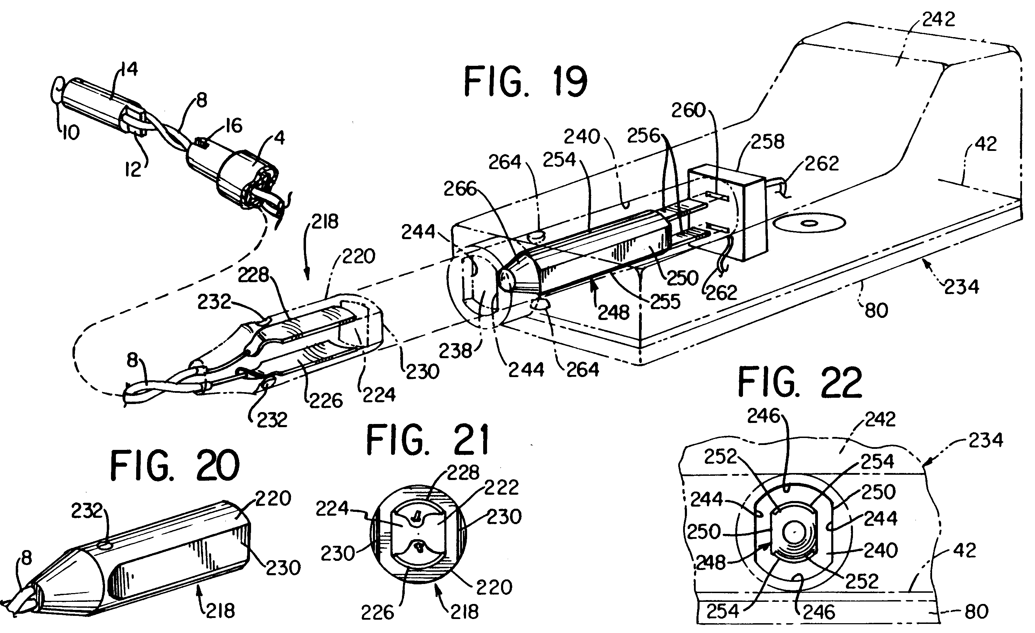

Figures 19-24 show various embodiments of female connector and leg plate assemblies in accordance with the present invention. The leg plate, shown in outline only, is identical to any of the previously mentioned embodiments of leg plates which contain a printed circuit board, except that, instead of barrel contacts, a male connector is employed. Also, the twisted wire pair with fetal and maternal electrodes is identical to that previously described.

Each of the female connectors of the embodiments of Figs. 19-24 have an external diameter which is less than the inside diameter of the driving tube. Since the outer diameter of the guide tube 4 must be small enough to avoid harming the mother during its insertion, an outer diameter of about 0.315 inches is acceptable; the driving tube 6 and the female connector must have even smaller external diameters to fit in the guide tube.

Figure 19 shows the female connector 218 having a hollow rod-shaped housing 220 in which a passage 222 extends from an open end 224. The passage 222 has a cross-section defined by two vertical sides and two convexly curved sides which extend between the vertical sides. One electrode is in electrical connection with the fetal (spiral) electrode and the other is in electrical connection with the maternal (spade) electrode via respective wires of the twisted wire pair 8. Two contacts 226, 228 are recessed back from the open end 224 and face each other within the passage 222.

Figures 20 and 22 show that the housing 220 has two outwardly facing rod-shaped flats 230 each on a respective side of the housing 220. The housing 220 also has a pair of detents 232 which are recesses in the external surface of the housing.

Figure 19 also shows the leg plate 234, which contains the printed circuit board (PCB) 42. The leg plate has an opening 238 which communicates with an internal passage 240 within the housing 242 of the leg plate. The housing 242 defines the opening 238 by two oppositely facing flat walls 244 and two oppositely facing concavely curved walls 246. The internal periphery of the opening 238 matches the external shape of the housing 220 of the female connector 218 (compare figures 21 and 22). This allows insertion of the female connector 218 into the opening 238 in a predetermined relative orientation to ensure that the electrical connection is established with both contacts of a male connector 248.

The male connector 248 extends inside the passage 240 and has flat sides 250 in alignment with the flat walls 244 of the opening 238. The male connector 248 also has two convexly curved sides 252 which extend between the flat sides 250 and on which is a respective conductive contact 254, 255. This conductive contact may be a metallic plate or coating. Two plug terminals 256 extend from an end of the male connector 248 and each is in electrical connection with a respective one of the conductive contacts 254.

A vertically extending bulkhead or body 258 is mounted (such as by soldering) on top of the PCB 42 and has two socket terminals 260, into which may be inserted the two plug terminals 256 simultaneously for establishing electrical connection. Thus, the male connector 248 is removable from the bulkhead 258 and thereby from the leg plate.

The PCB 42 has two input terminals 114A, 114B as shown in Fig. 6. The two input terminals are connected to the two outlet terminals 260 via respective leads 262. Protrusions 264 extend inwardly within the passage 240 and are configured to engage with the detents 232 of the female connector 218 to releasably retain the female connector 218 to the male connector 248.

After plugging the male connector 248 into the socket terminals 260 of the bulkhead 258, the male connector is ready to be inserted into the female connector 218. This requires that the female connector 218 be inserted into the passage 240 as far as possible. In order to ensure that electrical connection is effected between the contacts, the female connector 218 may be inserted through the opening 238 only when the flat sides 230 are in alignment against the flat walls 244 of the leg plate housing 242.

The female connector 218 is inserted onto the male connector 248, essentially shrouding or enclosing the same. The male connector 248 has a forward end 266 which is tapered to facilitate insertion of the male connector into the opening 224 of the female connector 218. The contacts 226, 228 resiliently bias in response to insertion of the male connector 248 within the passage 222 so as to maintain electrical connection between the contacts 226, 254 and 228, 255. Thus, when inserting, the tapered forward end 266 eventually pushes the contacts 226 outward so that they exert a biasing force against the contact 254 thereafter.

The female connector 218 is inserted until the protrusions 264 and detents 232 engage each other, at which the connectors 218, 248 snap together by a holding force which tends to resist inadvertent separation, i.e., an intentional manual pulling force is required to effect separation. Further insertion of the female connector 218 relative to the male connector 248 beyond this protrusion/detent engagement is prevented by the bulkhead 258, which is in a path to block such further insertion.

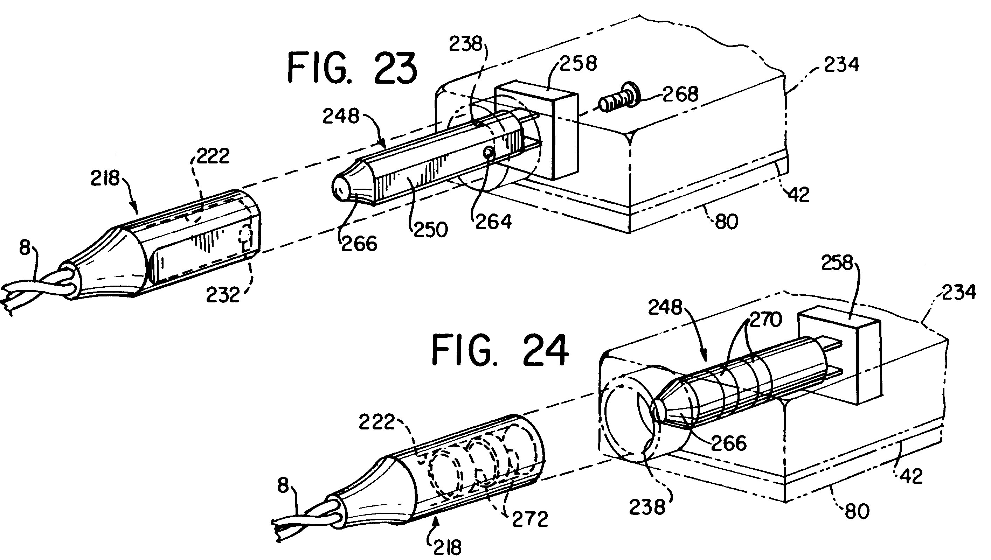

Fig. 23 shows another embodiment in which the male connector 248 protrudes through the opening 238 because the bulkhead 258 is positioned closer to the opening 238 than it was for the embodiment of Fig. 19. The protrusions 264 each lie on a respective one of the flat sides adjacent the opening 238 and the detents 232 are almost adjacent to the forward end of the female connector 218. When inserting the male connector 248 within the passage 222 of the female connector, the female connector 218 shrouds or encloses the male connector 248 until engagement between the protrusions and detents is effected. Instead of the bulkhead 258 effecting blocking, a portion of the leg plate housing 242 immediately adjacent the opening 238 may serve to block further insertion of the female connector onto the male connector.

Also, the male connector 248 may have leads soldered to terminals of the bulkhead 258 and be fastened to the bulkhead 258 by means of a screw 268 or other fastener. The bulkhead 258 may be soldered to the PCB 42 or be screwed into threaded holes (not shown) in the PCB 42. The leg plate, printed circuit board, female connector, twisted wire pair and electrodes are otherwise the same as that of Fig. 19.

Fig. 24 more closely resembles the embodiment of Fig. 4 by employing ring contacts 270 on the male connector and barrel contacts 272 within the passage 222 of the female connector 218. These connectors 218, 248 are otherwise identical to any of the other previously described embodiments. An advantage of the ring type contacts is that no particular orientation configuration is needed. Like the other embodiments, a detent/protrusion engagement may be employed to releasably lock the connectors together. The bulkhead 258 also blocks further relative insertion of the female connector onto the male connector beyond that which would result in disconnection of the electrical connection between the ring contacts and barrel contacts.

The male connector of Fig. 24 may project instead out of the leg plate housing in the same manner in which the male connector of Fig. 23 projects out of the leg plate housing, so as to provide for a further embodiment. The female connector shrouds the male connector in the same manner to effect electrical connection.

The connectors 218, 248 of any embodiment may have any shape. When one connector shrouds the other, some surfaces face each other. Preferably, such surfaces complement each other in shape for effecting engagement to ensure maintenance of electrical contact therebetween. It does not make a difference which contact of any female connector electrically connects with which leg plate contact so long as both contacts of the female connector are in electrical connection.

The detents and protrusions of each embodiment may be exchanged with each other, i.e., the female connector may have the protrusions and the male connector of the leg plate may have the detents or vice versa. Instead of engaging with separate detents/protrusions, the contacts themselves may provide the same function by having at least one contact of one connector located in a recess in such connector and by having a corresponding contact of another connector bias into the recess after it reaches the recess during the insertion. Any other means of releasably retaining the male and female connectors together, such as by friction fitting, may be used instead of detents and protrusions.

Further, where any embodiment shows various components for accomplishing the same task accomplished by different components of another embodiment, the components are interchangeable between the embodiments. For instance, any embodiment may employ a bulkhead to secure its connector to the printed circuit board or leg plate housing. The bulkhead and connector may be secured together by plug/socket engagement as in Fig. 19 or by screw engagement as in Fig. 23. In short, the manner of securement of the male or female connector to the leg plate of any embodiment may replace the manner of securement described any of the other embodiments.

While the foregoing description and drawings represent the preferred embodiments of the present invention, it will be understood that various changes and modifications may be made without departing from the spirit and scope of the present invention.

## Claims

1. A connector device for electrically connecting fetal and maternal electrodes to a plate support (30) on the body of an expectant mother, comprising:

   a housing (32) of the plate support;

   electrical connection means for making electrical connection between a remote fetal monitoring device and the fetal and maternal electrodes (10, 12), the connection means including input and output means(50A, 50B)which are electrically connected together within the housing, the input means including a male connector (20, 248) and a female connector (38, 40, 218) adapted to engage each other for establishing electrical connection therebetween, the one of the male connector and the female connector being electrically connected with the fetal and maternal electrodes, the other of the male connector and the female connector being electrically connected with the output means, the output means being adapted for making electrical connection with the remote fetal monitoring device; and

   releasable retaining means (38B, 44, 232, 264) for releasably retaining the male and female connectors together when the electrical connection is established between the male and female connectors.

2. A device for electrically connecting fetal and maternal electrodes with a plate support on the body of an expectant mother, comprising:

   a housing (32) of the plate support;

   electrical connection means for making electrical connection between a remote fetal monitoring device and the fetal and maternal electrodes (10, 12), the connection means including a male or female connector and an output means which are electrically connected together within the housing, the male or female connector having two contacts (38, 40) electrically isolated from each other and in electrical connection with the output means said male or female connector being adapted to make slidable and releasable electrical connect ion with the fetal and maternal electrodes, the output means being adapted for making electrical connection with the remote fetal monitoring device.

3. A device as claimed in Claim 1 or Claim 2, further characterized by:

   a base (76, 86);

   a ground electrode (78) supported by the base; means for electrically connecting the output means (50C) with the ground electrode and for securing the base and housing together; and

   means for securing at least one of the base and the housing to the mother so that the ground electrode is in electrical connection with the mother.

4. A device as claimed in Claim 3, characterized in that the attaching means includes means for enabling the housing to rotate relative to the base.

5. A device as claimed in Claim 4, characterized in that the housing has sides with corners, further comprising means for blocking the housing from rotating relative to the base by acting against the corners.

6. A device as claimed in any one of Claims 1 to 5 Claim 1, characterized in that one of the male and female connectors has two terminals electrically isolated from each other and each in electrical connection with a respective one of the fetal and maternal electrodes.

7. A device as claimed in any one of Claims 1 to 6 characterized in that the housing (32) has an internal passage in which is located the input means and into which the one of the male and female connector means is insertable and removable.

8. A device as in Claim 7, characterized in that the internal passage has an entrance (34) and a chamber (142) adjacent the entrance, further comprising means (144) arranged in the chamber for wiping the one of the male and female connector means clean and for sealing the chamber from the outside during insertion of the one of the male and female connector means into the passage while passing by the chamber.

9. A device as claimed in any one of Claims 1 to 8 further characterized by:

   a printed circuit board within the housing, the printed circuit board having conductive tracers which are part of the electrical connection means.

10. A device as claimed in Claim 9, characterized by a bulkhead (258) extending from the printed circuit board, the bulkhead and the other of the male and female connectors having detachable engaging elements which engage and are in electrical connection with each other as part of the electrical connection means.

11. A device as claimed in Claim 9 or Claim 10, further characterized by:
conductive test terminals (48A, 48B) which are accessible from outside the housing, the test terminals being in electrical connection with respective ones of the conductive tracers.

12. A device as claimed in Claim 11 further characterized by:
an adapter (124) having terminals arranged to be in electrical connection with the conductive test terminals.

13. A device for electrically connecting fetal and maternal electrodes with a plate support on the body of an expectant mother, comprising:
a housing (32) of the plate support;
electrical connection means for making electrical connection between a remote fetal monitoring device and the fetal and maternal electrodes (10, 12), the connection means including a connector and an output means which are electrically connected together within the housing, the connector having two contacts (38, 40) electrically isolated from each other and in electrical connection with the output means, the output means being adapted for making electrical connection with the remote fetal monitoring device;
a base (76, 78);
a ground electrode (78) supported by the base;
means for electrically connecting the output means with the ground electrode and for securing the base and housing together; and
means for securing at least one of the base and the housing to the mother so that the ground electrode is in electrical connection with the mother.

14. A device for electrically connecting fetal and maternal electrodes to a plate support on the body of an expectant mother, the plate support having a housing with a male or female connector (38, 40, 248), the device comprising:
fetal (10) and maternal (12) electrodes; and

electrical connection means for making electrical connection between a remote fetal monitoring device and the fetal and maternal electrodes, the connection means including a connector (20, 218) electrically connected with the fetal and maternal electrodes, the connector having a body with two contacts (24, 26, 226, 228, 272) electrically isolated from each other, each of the contacts being in electrical connection with a respective one of the electrodes.

15. A device as in Claim 14, further comprising a hollow handle (17) secured to a driving tube (6), the handle widening in diameter as the handle extends away from the driving tube, the connector (20, 218) having a tapered forward end (16) insertable into the hollow of the handle to avoid jamming.

Fig.1.

Fig.2.

Fig.3.

Fig. 5.

111

38B
38A
28
22
38A
38B

38
38A
5
40
40A
40B
112B
48A
114c
48C
112A
18
24
44
5
28
22
20
5
110A
110B
48B
42
72

Fig. 4.

114B
118
98B
42
98A
120
94
102B
122
104
106
102A
64
98C
100
116
114c
96B
98A
98C

Fig. 6.

114A

EP 0 484 107 A1

Fig.7.

Fig.8.

Fig.9.

Fig. 13.

Fig. 11.

Fig. 10.

Fig. 12.

Fig.14.

Fig.15.

EP 0 484 107 A1

## Fig.16.

## Fig.17.

# FIG. 18

FIG. 19

FIG. 20

FIG. 21

FIG. 22

EP 0 484 107 A1

FIG. 23

FIG. 24

### European Patent Office

## EUROPEAN SEARCH REPORT

Application Number

EP 91 30 9987

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | DE-U-8 603 542 (DR. J. MÜLLER-WICKOP) <br><br>* page 10, line 04 - page 11, line 17; figures 1-3 * <br>--- | 1,2,13, 14 | A61B5/0448 <br> A61B5/0416 <br> H01R13/66 <br> A61B5/0428 |
| A | DE-U-8 701 828 (DR.J.MÜLLER-WICKOP) <br><br>* page 13, line 27 - page 14, line 14; figures 1-4 * <br>--- | 1,2,13, 14 | |
| A | EP-A-0 390 400 (LECTEC CORPORATION) <br><br>* abstract; figures 1-7 * <br>--- | 1,2,13, 14 | |
| A | EP-A-0 377 432 (ABBOTT LABORATORIES) <br> * abstract; figures 1-8 * <br>--- | 14,15 | |
| A | US-A-3 580 242 (G.E.LA CROIX) <br><br>* the whole document * <br><br>----- | 1,2,13, 14 | |
|  |  |  | TECHNICAL FIELDS SEARCHED (Int. Cl.5) <br><br> A61B <br> H01R |

The present search report has been drawn up for all claims

| Place of search | Date of completion of the search | Examiner |
|---|---|---|
| THE HAGUE | 05 FEBRUARY 1992 | HUNT B.W. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)